# EUROPEAN PATENT APPLICATION

(11) **EP 3 954 415 A1**
(43) Date of publication of application: **16.02.2022**
(21) Application number: 20787872.9
(22) Date of filing: 27.03.2020
(51) Int. Cl.: A61M 11/00, B05B 17/06

(54) **AEROSOL SUPPLY DEVICE**

(30) Priority: 09.04.2019 JP 2019074360
(71) Applicant: Japan Tobacco Inc., Tokyo 105-6927 (JP)
(72) Inventor: INAGAKI, Michihiro, Tokyo 130-8603 (JP); ABE, Yuki, Tokyo 130-8603 (JP); WAKAMATSU, Miki, Tokyo 130-8603 (JP); SUGANUMA, Tatsuya, Tokyo 130-8603 (JP)
(74) Representative: Hoffmann Eitle
(86) International application number: PCT/JP2020/014314
(87) International publication number: WO 2020/209111

(57) **Abstract**

An aerosol supply device includes: an accommodating part (cartridge (4)) configured to accommodate a liquid (10); an atomization unit (5) configured to atomize the liquid (10) to generate an aerosol (11); a discharge port (3a) configured to discharge the generated aerosol (11) to the outside of the aerosol supply device; and a volatilization preventing part (solenoid valve (8a) or duckbill valve (8b)) capable of opening and closing a passage (first passage (6) or second passage (7)) through which at least one of the liquid (10) or the aerosol (11) flows between the accommodating part and the outside of the aerosol supply device.

## Description

### Technical Field

The present invention relates to an aerosol supply device, which is configured to supply an aerosol to the outside of the aerosol supply device.

### Background Art

Aerosol supply devices such as flavor inhalers capable of delivering flavor without combustion of a flavor source such as tobacco are now in widespread use. Other known examples of aerosol supply devices include inhalers capable of atomizing a liquid by using ultrasonic waves for supply to the user.

For instance, PTL 1 discloses an aerosol supply device (referred to as inhalation thermotherapy apparatus in PTL 1) in which water is sufficiently heated and dispersed, and atomized via an injection nozzle and a conduit into an aerosol for supply into the nasal cavity through a suction port (referred to as suction component in PTL 1).

The aerosol supply device according to PTL 1 is specifically configured as described below. The base plate of an accommodating part is provided with a heat conduction plate, with a heater disposed on the lower surface of the heat conduction part so that even if the amount of water within the accommodating part (referred to as injection tank in PTL 1) decreases, the water can be sufficiently heated.

### Citation List

### Patent Literature

PTL 1: Japanese Unexamined Utility Model Registration Application Publication No. 4-131223

### Summary of Invention

### Technical Problem

A potential problem with the aerosol supply device according to PTL 1 is that because the interior of the accommodating part is always exposed to the outside through the suction port, if the aerosol supply device is left unused for an extended period of time, water accommodated within the accommodating part may volatilize and decrease in quantity in some cases.

If, for instance, a liquid containing even the slightest amount of solute is used to supply an aerosol, volatilization of moisture may cause the concentration of the liquid to change in some cases.

Further, there are cases where volatilization of moisture causes precipitation of a water mixture, which clogs the conduit used for atomization.

The present invention has been made in view of the above-mentioned problems. Accordingly, it is an object of the present invention to provide an aerosol supply device capable of preventing volatilization of a liquid.

### Solution to Problem

An aerosol supply device according to the present invention includes: an accommodating part configured to accommodate a liquid; an atomization unit configured to atomize the liquid to generate an aerosol that is to be supplied to an outside of the aerosol supply device; and a volatilization preventing part capable of opening and closing a passage through which the liquid or the aerosol flows between the accommodating part and the outside.

### Advantageous Effects of Invention

The aerosol supply device according to the present invention makes it possible to reduce volatilization of a liquid within an accommodating part.

### Brief Description of Drawings

[Fig. 1] Fig. 1 schematically illustrates the configuration of an aerosol supply device according to a first embodiment.
[Fig. 2] Fig. 2 schematically illustrates a liquid being atomized and discharged to the outside of the aerosol supply device.
[Fig. 3] Fig. 3 is a plan view of an atomization unit.
[Fig. 4] Fig. 4 schematically illustrates duckbill valves in those closed state when the aerosol supply device is not in use.
[Fig. 5] Fig. 5 schematically illustrates the internal configuration of a device body of an aerosol supply device according to a second embodiment.
[Fig. 6] Fig. 6 schematically illustrates duckbill valves according to a first modification.
[Fig. 7] Fig. 7 schematically illustrates a volatilization preventing part according to a second modification, of which Fig. 7(a) illustrates the volatilization preventing part with a storing part exposed, and Fig. 7(b) illustrates the volatilization preventing part when hermetically sealing an area in the vicinity of the storing part.

### Description of Embodiments

Embodiments of the present invention are described below with reference to the drawings. The following description of the embodiments is illustrative only for purposes of better understanding the present invention, and not to be taken as limiting the present invention. It is to be readily appreciated, therefore, that the shapes, dimensions, arrangements, or other details of components or parts described below may be changed or modified without departing from the scope of the present invention, and that all such equivalents fall within the scope of the present invention.

Throughout the drawings, like components are designated by like reference signs, and overlapping descriptions are omitted as appropriate.

### <Overview>

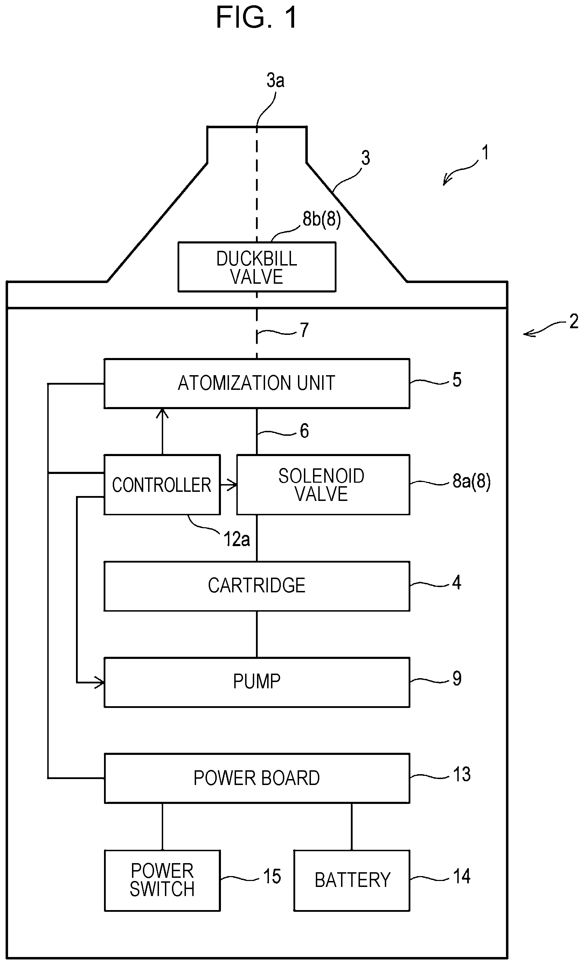

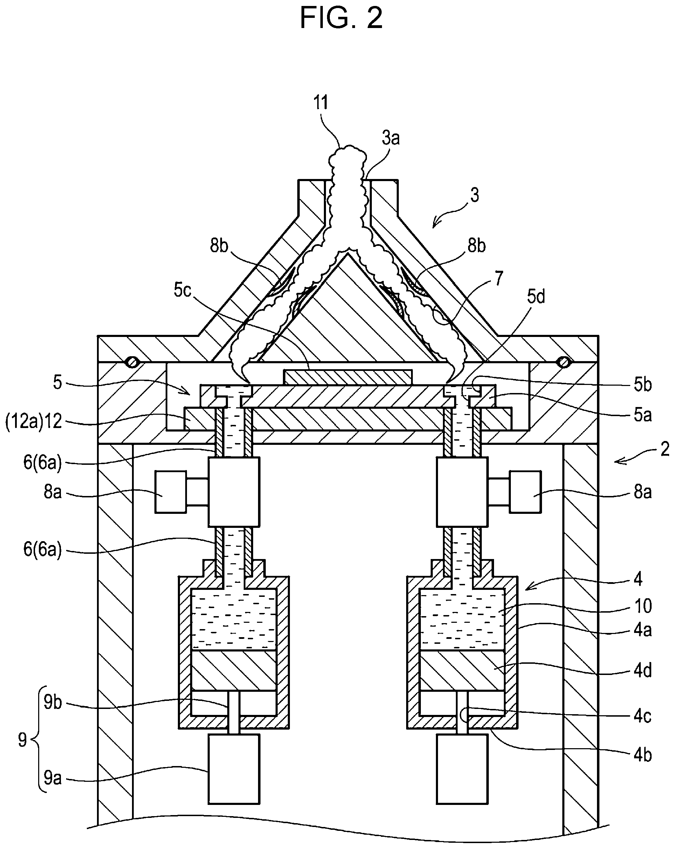

An overview of an aerosol supply device 1 according to the embodiments is first described below with reference to Figs. 1 and 2. Fig. 1 schematically illustrates the configuration of the aerosol supply device 1 according to a first embodiment. Fig. 2 schematically illustrates a liquid 10 being atomized and discharged to the outside of the aerosol supply device 1. Although gaskets or other packing materials are provided between various components of the aerosol supply device 1 from the viewpoint of ensuring hermetic sealing, some of such packing materials are not depicted in Fig. 2 or other figures to facilitate understanding of the present invention.

The aerosol supply device 1 according to the embodiments includes an accommodating part (a cartridge (4)), an atomization unit 5, a discharge port 3a, and a volatilization preventing part 8 (at least one of a solenoid valve 8a or a duckbill valve 8b). The accommodating part is configured to accommodate the liquid 10. The atomization unit 5 is configured to atomize the liquid 10 to generate an aerosol 11. The discharge port 3a is configured to discharge the generated aerosol 11 to the outside of the aerosol supply device 1. The volatilization preventing part 8 is capable of opening and closing a passage through which at least one of the liquid 10 or the aerosol 11 flows between the accommodating part (cartridge 4) and the outside of the aerosol supply device 1.

According to the configuration mentioned above, the volatilization preventing part 8 is capable of opening and closing the passage through which the liquid 10 or the aerosol 11 flows between the accommodating part (cartridge 4) and the outside of the aerosol supply device 1. Therefore, by closing the passage, volatilization of the liquid 10 within the accommodating part, in other words, flowing out of the aerosol 1 1 to the outside of the aerosol supply device 1 can be reduced. Reducing volatilization of the liquid 10 within the accommodating part also makes it possible to prevent changes in the concentration of the liquid 10 within the accommodating part.

### «First Embodiment»

### <Configuration of Various Parts>

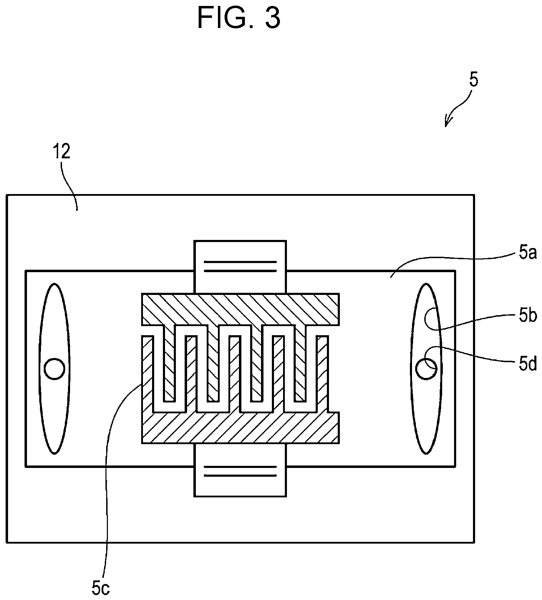

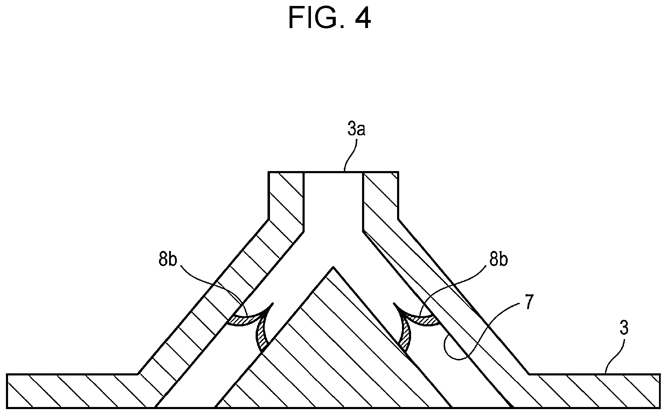

The configuration of various parts of the aerosol supply device 1 is now described below with reference to Figs. 3 and 4 in addition to Figs. 1 and 2. Fig. 3 is a plan view of the atomization unit 5. Fig. 4 schematically illustrates the duckbill valves 8b in those closed state when the aerosol supply device 1 is not in use.

### (Atomization Unit)

As illustrated in Figs. 2 and 3, the atomization unit 5 includes a substrate (piezoelectric substrate 5a), and an electrode 5c. The piezoelectric substrate 5a includes storing parts 5b to store the liquid 10, and a piezoelectric element. The electrode 5c is disposed in contact with the piezoelectric substrate 5a, and configured to supply a surface acoustic wave to the liquid 10 stored in the storing parts 5b disposed on the piezoelectric substrate 5a.

### (Piezoelectric Substrate)

The storing part 5b provided to the piezoelectric substrate 5a according to the first embodiment is a closed-end groove formed on the top surface of the piezoelectric substrate 5a and having an elliptical shape in plan view as illustrated in Fig. 3. The storing part 5b is disposed in at least two places, with the electrode 5c being disposed between the two places. The storing part 5b has a through-hole 5d provided in the central portion such that the through-hole 5d extends through the thickness of the piezoelectric substrate 5a. The storing part 5b receives the liquid 10 that is supplied from the cartridge 4 via a first passage 6 (tube 6a) and the through-hole 5d by means of a pump 9 described later.

### (Electrode)

The electrode 5c is a comb-shaped electrode (also referred to as interdigital transducer (IDT)). High-frequency electric power input from a power board 13 to the electrode 5c via a control board 12 is converted into a surface acoustic wave (also referred to as SAW) due to the piezoelectricity of the piezoelectric substrate 5a, and propagates along the surface of the piezoelectric substrate 5a on both sides in the direction of arrangement of the comb teeth.

The number of comb teeth of the electrode 5c is not limited to the number depicted but is determined based on the efficiency of atomization by which the aerosol 11 is produced by using a surface acoustic wave. The width and spacing of the comb teeth are determined based on a frequency based on the particle size of the aerosol 11 to be produced by atomization.

An important consideration with regard to a surface acoustic wave is the control of the frequency of electric power. Accordingly, sensors such as a resonant-frequency monitoring sensor for monitoring resonant frequency, or a temperature sensor (not illustrated) capable of detecting the temperature of the liquid 10 immediately before atomization may be provided.

The exterior construction of the aerosol supply device 1 according to the first embodiment mainly includes a device body 2, and a mouthpiece 3 attached to an upper portion of the device body 2 and through which the aerosol 11 is to be discharged.

### (Mouthpiece)

The mouthpiece 3 according to the first embodiment is formed such that, in front view, the mouthpiece 3 extends to be wider in an end portion near the bottom, and has a tapered part with a width that decreases toward the discharge port 3a located at the top end of the mouthpiece 3 and through which the aerosol 11 is to be discharged externally.

As illustrated in Fig. 2, the mouthpiece 3 has a passage (second passage 7) through which to pass the aerosol 11 generated from the atomization unit 5. The passage (second passage 7) includes two separate passages each disposed to allow passage of the aerosol 11 generated from the liquid 10 stored in the storing parts 5b disposed in the at least two places. The two second passages 7 are formed symmetrically with respect to an imaginary plane passing through the discharge port 3a. The two second passages 7 join into a single passage immediately before reaching the discharge port 3a located at the top end of the mouthpiece 3.

The separate passages (second passages 7) may be formed to have different lengths. In other words, the two second passages 7 may not necessarily be formed symmetrically with respect to the imaginary plane passing through the discharge port 3a but may be formed asymmetrically. For example, if the two second passages 7 allow passage of aerosols 11 containing different components, each passage may be formed with a length or shape appropriate to the corresponding aerosol 11.

In a particularly preferred example, such separate second passages 7, for example, two separate second passages 7 each include an asymmetric portion formed with a different curvature.

According to the above-mentioned configuration, by bringing the aerosol 11 into contact with a wall surface defining each of the asymmetric portions of the two second passages 7 with different curvatures, particles contained in the aerosol can be deposited/reduced, and their particle size can be adjusted. This allows the user to smoothly inhale the aerosol 11 without experiencing discomfort in the throat caused by components contained in the aerosol 11.

The term "curvature" as used herein is meant to conceptually include not only sharp bends but also gentle bends. Examples of two second passages 7 with different curvatures include not only two passages that are both bent but also two passages including one straight passage.

### (Device Body)

The following components are disposed inside the device body 2 according to the first embodiment: the cartridge 4; the atomization unit 5; the volatilization preventing part (solenoid valve 8a) configured to open and close the first passage 6, described later, through which the liquid 10 flows; the pump 9 configured to supply the liquid 10 from the cartridge 4 to the atomization unit 5; the control board 12 including a controller 12a configured to control various electronics; and the power board 13, a battery 14, and a power switch 15 exposed to the outside of the device body 2, which are illustrated in Fig. 1.

In the device body 2, a passage (first passage 6) for the liquid 10 is provided between the accommodating part (cartridge 4), which will be described later, and the atomization unit 5. Further, a passage (second passage 7) for the aerosol 11 is provided between the atomization unit 5 and the outside of the device body 2 so as to extend from the device body 2 to the mouthpiece 3.

### (Cartridge)

The cartridge 4 according to the first embodiment includes a body part 4a, a base plate 4b, and a sliding block 4d. The base plate 4b is integrated with the bottom portion of the body part 4a. The sliding block 4d is disposed inside the cartridge 4, and arranged in a watertight manner on the inner wall surface of the body part 4a. The sliding block 4d is capable of sliding within the body part 4a.

The base plate 4b has a through-hole 4c extending through the thickness of the base plate 4b. A push rod 9b of the pump 9, which will be described later, can be inserted into the through-hole 4c.

According to the first embodiment, the liquid 10 can be supplied separately from each of two cartridges 4 to the corresponding one of two storing parts 5b provided to the piezoelectric substrate 5a of the atomization unit 5.

However, the present invention is not limited to this configuration. Alternatively, the liquid 10 may be supplied from a single cartridge 4 to two storing parts 5b. This configuration makes it possible to reduce the number of solenoid valves 8a described later. That is, the solenoid valve 8a may be simply provided to a portion of the tube 6a near the cartridge 4, at a position before where the tube 6a splits in two toward the two storing parts 5b.

The above-mentioned configuration makes it possible to, by means of a single solenoid valve 8a, control whether to supply the liquid 10 to two storing parts 5b or stop the supply of the liquid 10.

The aerosol supply device 1 may further include a plurality of cartridges 4. If two or more cartridges 4 are to be used, individual cartridges 4 may accommodate liquids 10 with different components such as taste or fragrance.

The accommodating part according to the present invention may not necessarily be the cartridge 4 that is removable, but may be a container pre-installed on the device body 2 (i.e., a refillable container). If such a pre-installed container is to be used, the container may be filled with the liquid 10 in a similar manner. That is, another passage communicating with the container and the outside may be formed in advance, and the liquid 10 may be charged into the container through this passage to fill the container with the liquid 10.

The above-mentioned configuration eliminates the need for the user to, when refilling the container with the liquid 10, remove pre-installed components including the solenoid valve 8a, the cartridge 4, and the tube 6a connecting the solenoid valve 8a and the cartridge 4, and attach these components again.

### (Liquid)

The liquid 10 may include a solvent such as water, glycerol, propylene glycol, or ethanol. Further, the liquid 10 may include a solute containing a component such as taste or fragrance. Exemplary solvents include volatile components such as tobacco extracts, menthol, linalool, limonene, or vanillin, and non-volatile components, for example, sugars such as fructose, glucose, sucrose, or lactose, acids such as malic acids or citric acids, or salts.

The liquid 10 may be emulsified by an emulsifier, or may be suspended by a dispersant.

### (Volatilization Preventing Part)

The volatilization preventing part 8 (solenoid valve 8a) is connected to the tube 6a that connects the cartridge 4 and the atomization unit 5 within the device body 2.

The volatilization preventing part 8 (solenoid valve 8a) includes an open/close part (not illustrated) capable of opening and closing the passage (first passage 6) through which the liquid 10 flows.

According to the configuration mentioned above, by the controller 12a operating the open/close part of the solenoid valve 8a to close the first passage 6, volatilization of the liquid 10 from the accommodating part (cartridge 4) can be prevented. Further, by the controller 12a operating the open/close part of the solenoid valve 8a to open the first passage 6, the liquid 10 can be supplied to the atomization unit 5, and consequently the aerosol 11 can be supplied to the user.

In the following description of the first embodiment, the duckbill valves 8b constituting a check valve are described as an example of the volatilization preventing part 8 configured to open and close the passage for the aerosol 11. The duckbill valve 8b according to the first embodiment is a valve including opposed flat plates made of a soft resin material, with the spacing between the flat plates decreasing progressively toward the downstream (suction) side. The duckbill valve 8b is configured such that in normal condition, the flat plates contact each other to close the passage, and when a suction pressure is applied from the downstream side, the flat plates move away from each other to open the passage.

The duckbill valve 8b acts as a small check valve to allow the passage for the aerosol 1 1 to narrow. This makes it possible to reduce the size of the mouthpiece 3 and therefore the size of the aerosol supply device 1. The duckbill valve 8b is preferred for its ability to open by a small suction force without being affected by the direction of gravity, and for its ability to naturally close the open/close part due to the elastic restoring force once the suction stops.

The two second passages 7 of the mouthpiece 3 are each provided with the volatilization preventing part 8 (duckbill valve 8b).

The volatilization preventing part (duckbill valve 8b) is disposed in each of the two passages at an equal distance from the discharge port 3a.

According to the above-mentioned configuration, the aerosol 11 generated from the storing parts 5b disposed in two places can be discharged through the discharge port 3a at the same timing. This helps to reduce variations in the rate at which the aerosol 11 is discharged.

The volatilization preventing part 8 (duckbill valve 8b) is capable of transition between a state in which the volatilization preventing part 8 closes the passage (second passage 7) for the aerosol 11, and a state in which the volatilization preventing part 8 opens the passage (second passage 7) for the aerosol 11.

According to the above-mentioned configuration, closing the second passage 7 for the aerosol 11 by means of the duckbill valve 8b makes it possible to limit volatilization of the liquid 10 within a hermetically sealed space with the saturated vapor pressure of the liquid 10 as an upper limit to thereby inhibit precipitation of the solute on the atomization unit 5. This helps to reduce deterioration of atomization efficiency.

The volatilization preventing part 8 according to the first embodiment is a valve element (duckbill valve 8b) configured to close the passage (second passage 7) for the aerosol 11 in a first state, and open the passage (second passage 7) for the aerosol 11 in a second state in which the pressure condition has changed due to suction applied by the user. The volatilization preventing part (duckbill valve 8b) is disposed between the storing part 5b and the discharge port 3a.

More specifically, the "first state" according to the first embodiment refers to a state in which the air inside the aerosol supply device 1 is not being sucked by the user through the discharge port 3a. Conversely, the "second state" refers to a state in which the air inside the aerosol supply device 1 is being sucked by the user through the discharge port 3a.

According to the above-mentioned configuration, the open/close part is a valve element. Consequently, while suction is applied by the user, the aerosol 11 can be supplied to the user, and while suction is not applied by the user, the second passage 7 is closed to prevent volatilization of the liquid 10 to the external environment through the discharge port 3a.

Further, as described above, the aerosol supply device 1 that atomizes the liquid 10 by means of a surface acoustic wave includes the volatilization preventing part (duckbill valve 8b) disposed between the storing part 5b and the discharge port 3a. As a result, precipitation of the solute on the storing part 5b can be prevented. This helps to reduce variations in the concentration of the solute in the liquid 10, thus allowing stable taste or fragrance.

The "valve element" according to the present invention is not limited to the duckbill valve 8b. The valve element may be a solenoid valve similar to the solenoid valve 8a, an air-operated pinch valve with a tube closed by the pressure applied by supplied air, or another electrically driven valve. For example, if an electrically driven valve is to be used, a portion of the second passage 7 may be simply formed by a flexible tube to which the electrically driven valve can be connected.

By using an electrically driven valve instead of the duckbill valve 8b, the aerosol 11 can be discharged externally by the controller 12a controlling the opening action of the electrically driven valve, without the user having to suck on the discharge port 3a of the mouthpiece 3 and apply suction pressure. That is, an electrically driven valve is suited for use in diffusers that do not rely on the suction applied by the user to spray an aerosol. For example, the opening action of an electrically driven valve is controlled by the controller 12a in accordance with an operation performed on the power switch 15 as will be described later.

Although the duckbill valve 8b as a check valve has been described above as an exemplary "valve element", this is not meant to be limiting. The valve element may be any valve element capable of preventing volatilization due to its ability to close a passage in its normal state. For example, the valve element may be one capable of permitting backflow when positive pressure is being applied.

Likewise, the volatilization preventing part for opening and closing the first passage 6 is not limited to the solenoid valve 8a but may be an air-operated pinch valve with a tube closed by the pressure applied by supplied air, or another electrically driven valve.

In another example, the volatilization preventing part for opening and closing the first passage 6 or the second passage 7 may be one that is driven non-electrically. That is, the volatilization preventing part used may be one that causes the first passage 6 or the second passage 7 to be opened and closed by a physical drive action (movement of a barrier) (not illustrated) responsive to the user attaching the mouthpiece 3 to the device body 2 or pushing the mouthpiece 3 into the device body 2.

With no suction pressure applied, as illustrated in Fig. 4, the duckbill valves 8b close the second passage 7. This helps to prevent foreign matter from passing into the atomization unit 5 even if the foreign matter enters the aerosol supply device 1 through the discharge port 3a when the aerosol supply device 1 is not in use. Discharge of the aerosol 11 containing such foreign mater can be thus reduced, which proves advantageous also from the viewpoint of sanitation.

### (Pump)

The aerosol supply device 1 further includes the pump 9 capable of supplying the liquid 10 to the atomization unit 5. The pump 9 according to the first embodiment is a syringe pump capable of pulling the liquid 10 back toward the accommodating part (cartridge 4) from the atomization unit 5.

Although described in more detail later, using a syringe pump as the pump 9 makes it possible to pull the liquid 10 back toward the accommodating part (cartridge 4) from the atomization unit 5.

By thus reducing the amount of the liquid 10 remaining on the atomization unit 5, precipitation of the solute on the atomization unit 5 can be reduced.

More specifically, the pump 9 includes a motor 9a, and the push rod 9b capable of advancing or retracting as the motor 9a rotates. As the pump 9 operates, the push rod 9b passes through the through-hole 4c and pushes in the sliding block 4d upward. At this time, the liquid 10 is pushed in by the sliding block 4d and supplied to the atomization unit 5.

If the pump 9 is a syringe pump as described above, by retracting the push rod 9b from the through-hole 4c of the cartridge 4, the liquid 10 can be pulled in the reverse direction. The liquid 10 can be thus pulled in to a position where the liquid 10 leaves the storing parts 5b. This configuration is preferred for the ability to reduce precipitation of the solute on the storing parts 5b.

For instance, there are cases where, if the liquid 10 contains citric acid mixed therein, the citric acid that has not turned into mist may precipitate out of the liquid remaining in the storing parts 5b and solidify. In this regard, by pulling the liquid 10 in the reverse direction from the storing parts 5b by means of the pump 9 as described above, precipitation of citric acid on the storing parts 5b is reduced to thereby reduce variations in the concentration of citric acid in the liquid 10. This allows stable taste or fragrance.

Further, the sliding block 4d that comes into contact with the liquid 10 is disposed in the cartridge 4. This means that the pump 9 (push rod 9b) does not directly contact the liquid 10. As a result, for instance, in changing different kinds of cartridges 4 to change the kind of the liquid 10 having taste or fragrance, even if the pump 9 is not replaced, mixing of components such as taste or fragrance do not occur before and after the changing of the cartridges 4.

However, the pump according to the present invention may not necessarily be configured as described above but may be, for example, a piezoelectric pump (not illustrated). Using a piezoelectric pump makes it possible to provide a product with low electric power consumption.

### (Control Board)

The aerosol supply device 1 includes the control board 12 having the controller 12a configured to control operations of components such as the pump 9 and the volatilization preventing part 8 (solenoid valve 8a).

The controller 12a according to the first embodiment changes the solenoid valve 8a from a closed state, which is a normal state, to an open state in response to receiving a signal transmitted from the power board 13 and indicating that the aerosol supply device 1 has been activated by the user operating the power switch 15.

The controller 12a controls the atomization unit 5 such that electric power is supplied to the electrode 5c to generate a surface acoustic wave on the surface of the piezoelectric substrate 5a.

Further, the controller 12a activates the motor 9a of the pump 9 to cause the push rod 9b to push in the sliding block 4d in a direction (upward) that diminishes the volume of the cartridge 4. Consequently, the liquid 10 is supplied to the storing parts 5b through the first passage 6. The liquid 10 is thus resonated and atomized by a surface acoustic wave on the surface of the piezoelectric substrate 5a.

In this state, as the user sucks in the air inside the aerosol supply device 1 through the discharge port 3a, the pressure in a portion of the duckbill valve 8b near the discharge port 3a becomes negative. This causes the duckbill valve 8b to open, which allows the user to suck in the aerosol 11 passing through the duckbill valve 8b.

In one example, to prevent the aerosol 11 from continuing to be generated, the controller 12a may be configured to, in response to a pressure in the space around the atomization unit 5 becoming greater than or equal to a predetermined pressure due to generation of the aerosol 11, stop the generation of the aerosol 11.

In one specific example, a negative pressure sensor 16 described later may be disposed within the space where the atomization unit 5 is disposed, and the controller 12a may be configured to, in response to a signal provided from the negative pressure sensor 16, stop the pump 9, or close the open/close part of the solenoid valve 8a to thereby close the first passage 6.

The controller 12a is configured to, when the aerosol supply device 1 becomes no longer used, for example, when the power switch 15 is switched off, operate the solenoid valve 8a (open/close part) to close the passage for the liquid 10.

In a particularly preferred example, the controller 12a is configured to, in closing the passage for the liquid 10 as described above, control the pump 9 such that the liquid 10 is supplied to the atomization unit 5 before the liquid 10 is pulled back toward the accommodating part (cartridge 4) from the atomization unit 5, and cause the open/close part (solenoid valve 8a) to close the passage for the liquid 10 supplied to the atomization unit 5.

According to the above-mentioned configuration, if the solute precipitates out on the atomization unit 5, the liquid 10 is supplied to the cartridge 4 to dissolve the solute again. Then, the liquid 10 is pulled back toward the cartridge 4 from the atomization unit 5. This allows the amount of solute remaining on the atomization unit 5 to be reduced in comparison to a case where the liquid 10 is pulled back toward the cartridge 4 without being first supplied to the atomization unit 5. This helps to further reduce precipitation of the solute on the atomization unit 5 (piezoelectric substrate 5a).

### <Second Embodiment>

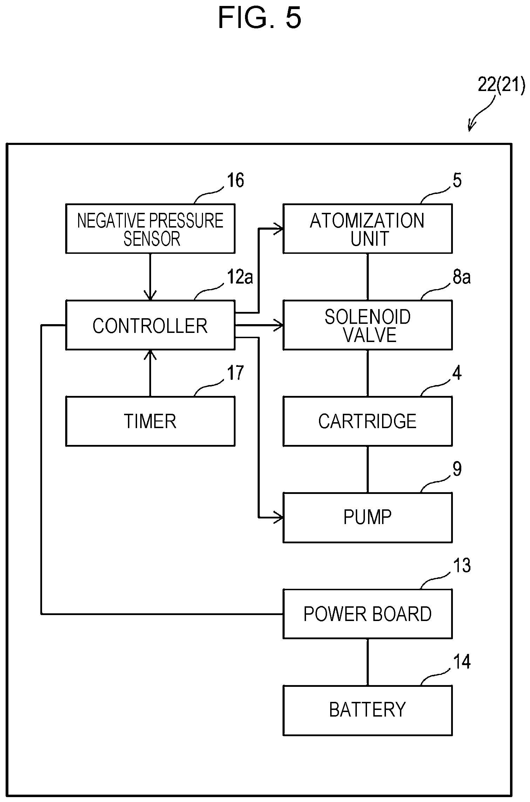

An aerosol supply device 21 according to a second embodiment is now described below with reference to mainly Fig. 5. Fig. 5 schematically illustrates the internal configuration of a device body 22 of the aerosol supply device 21 according to the second embodiment.

### (Negative Pressure Sensor)

The aerosol supply device 21 according to the second embodiment includes a detector (negative pressure sensor 16) disposed within a space where the atomization unit 5 is accommodated. The negative pressure sensor 16 is configured to detect a signal associated with suction of the aerosol 11 by the user.

The negative pressure sensor 16 includes a diaphragm, and a semiconductor strain gauge attached to the surface of the diaphragm. The negative pressure sensor 16 is a pressure sensor configured to convert, into an electrical signal, a change in electrical resistance due to the piezoelectric resistance effect caused by deformation of the diaphragm. The negative pressure sensor 16 may be any sensor capable of detecting a negative pressure generated by a user's puffing action. For example, the negative pressure sensor 16 may be a capacitive pressure sensor.

The controller 12a is configured to, in response to a signal transmitted from the detector (negative pressure sensor 16) and associated with the start of suction of the aerosol 11, control the volatilization preventing part (solenoid valve 8a) to deactivate a function of the volatilization preventing part (solenoid valve 8a) that prevents volatilization of the liquid 10.

By deactivating the volatilization preventing function of the volatilization preventing part (solenoid valve 8a) in response to a signal associated with the start of suction of the aerosol 11 as described above, the aerosol supply device 21 can be used smoothly.

The term "signal associated with suction" as used herein is meant to include not only a puff signal detected by the negative pressure sensor 16 but also a signal indicative of the conduction of electric power through the device body 2 at the time of suction (i.e., a signal based on the power switch 15 illustrated in Fig. 1).

The valve to be controlled by the controller 12a based on the negative pressure sensor 16 may not necessarily be the solenoid valve 8a but may be another electrically driven valve. Further, the electrically driven valve to be controlled by the controller 12a may not necessarily be an electrically driven valve disposed in the first passage 6 for the liquid 10. If a valve disposed in the second passage 7 is an electrically driven valve as described above, the controller 12a may be configured to control this electrically driven valve.

### (Timer)

The aerosol supply device 21 may include a timer 17 configured to measure the time after the end of acquisition of a signal associated with user's suction.

The controller 12a may be configured to, in response to determining based on a signal from the timer 17 that a predetermined amount of time or more has elapsed since the end of puffing, activate a function of the volatilization preventing part (solenoid valve 8a) that prevents volatilization of the liquid 10 (in other words, close the open/close part of the solenoid valve 8a).

According to the configuration mentioned above, the volatilization preventing function of the volatilization preventing part (solenoid valve 8a) is activated in response to a signal associated with the stop of suction of the aerosol 11. This helps to effectively prevent volatilization of the liquid 10 within the aerosol supply device 21.

A mechanism used to prevent volatilization by closing the tube 6a connected to the cartridge 4 may be an openable movable clamp (not illustrated) located outside the tube 6a.

### [First Modification]

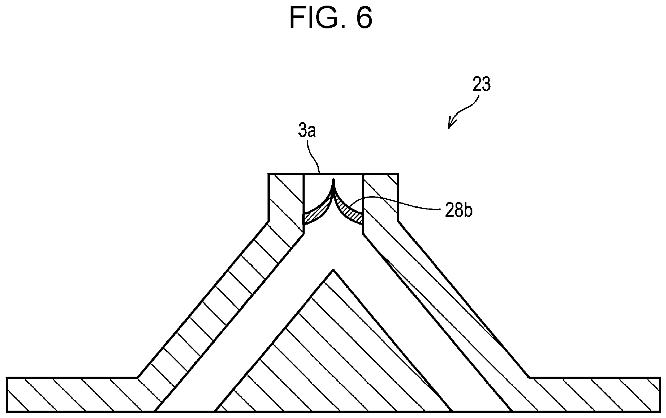

A mouthpiece 23 according to the first modification is now described below with reference to mainly Fig. 6. Fig. 6 schematically illustrates a duckbill valve 28b according to the first modification.

The volatilization preventing part 8 (duckbill valve 8b) according to the embodiments, which is disposed along the passage of the aerosol 11, is described above as being disposed in each of two second passages 7. Alternatively, however, as in the case of the mouthpiece 23 illustrated in Fig. 6, the volatilization preventing part 8 (duckbill valve 28b) may be disposed in a passage located downstream of where the two second passages 7 join.

The above-mentioned positioning eliminates the need to provide a plurality of duckbill valves 28b, and ensures that separate streams of the aerosol 11 do not join after being sparsely discharged from the two second passages 7. This helps to stabilize the amount of the aerosol 11 that is discharged through the discharge port 3a via the duckbill valve 28b.

### [Second Modification]

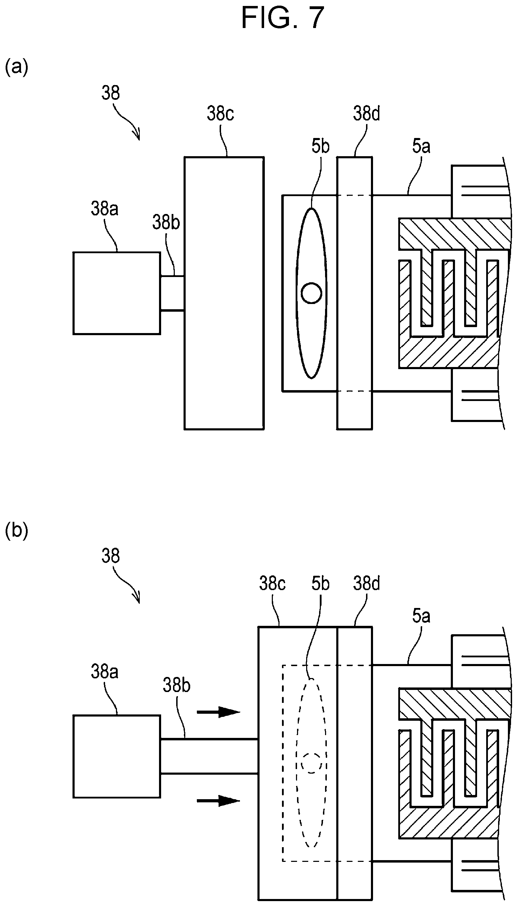

Lastly, a volatilization preventing part 38 according to a second modification is described below with reference to mainly Fig. 7. Fig. 7 schematically illustrates the volatilization preventing part 38 according to the second modification, of which Fig. 7(a) illustrates the volatilization preventing part 38 with the storing part 5b exposed, and Fig. 7(b) illustrates the volatilization preventing part 38 when hermetically sealing an area in the vicinity of the storing part 5b.

Although the foregoing description of the embodiments is directed to a configuration in which the duckbill valve 8b or 28b, or an electrically driven valve is used to open and close the passage (second passage 7) for the aerosol 11, the volatilization preventing part according to the present invention is not limited to the specific configuration mentioned above.

In another exemplary configuration, the volatilization preventing part 38 according to the second modification is capable of hermetically sealing the space in the vicinity of the storing part 5b (which is herein meant to include a case where the resulting space has a gap on the order of several microns).

Specifically, the volatilization preventing part 38 includes: a motor 38a; a push rod 38b configured to be actuated by the motor 38a so as to extend or contract; a sliding part 38c configured to make sliding movement due to the push rod 38b; and a stationary part 38d capable of forming a hermetically sealed space between the stationary part 38d and the sliding part 38c.

The motor 38a has a drive shaft (not illustrated) capable of being rotated in the forward or reverse direction by the controller 12a.

The push rod 38b is threadedly engaged with the drive shaft of the motor 38a. The push rod 38b is capable of advancing or retracting relative to the motor 38a as the motor 38a rotates.

The sliding part 38c has its lateral portion secured by the push rod 38b. A portion of the sliding part 38c with a square U-shaped cross section extending from the lateral portion and defined by the front, top, and back portions of the sliding part 38c extends in the direction of the axis of the push rod 38b.

The stationary part 38d is an upstanding wall disposed with a gap on the order of several micros between the stationary part 38d and the piezoelectric substrate 5a, and positioned transverse (orthogonal) to a straight line connecting the storing part 5b and the electrode 5c.

The controller 12a is configured to hermetically seal the space in the vicinity of the storing part 5b, in response to determining that the aerosol supply device 1 is no longer used, for example, in response to the timer 17 measuring the elapse of a predetermined amount of time after detection of a negative pressure by the negative pressure sensor 16.

More specifically, the controller 12a rotationally drives the motor 38a to advance the push rod 38b and press the push rod 38b against the sliding part 38c so that the sliding part 38c changes from the state illustrated in Fig. 7(a) to the state illustrated in Fig. 7(b) in which the sliding part 38c is in contact with the stationary part 38d.

Forming a hermetically sealed space as described above makes it possible to limit volatilization of the liquid 10 within this space with the saturated vapor pressure of the liquid 10 as an upper limit to thereby inhibit precipitation of the solute on the storing part 5b (atomization unit 5). This helps to reduce deterioration of atomization efficiency.

It is preferred if a completely hermetically sealed space can be created by the sliding part 38c and the stationary part 38d, because this helps to limit volatilization of the liquid 10. However, if such a configuration hinders propagation of SAW at a predetermined frequency, then as described above, a gap on the order of several microns small enough to prevent discharge of the liquid 10 may be provided between these parts and the piezoelectric substrate 5a.

The volatilization preventing part 38 may cover not only an area above the storing part 5b but also the entire area above the piezoelectric substrate 5a.

The aerosol supply device according to the present invention may not necessarily be an aerosol supply device that atomizes a liquid by using a SAW, but may be an aerosol supply device that atomizes a liquid by using, for example, a heating device such as a heater, or an ultrasonic wave oscillator.

Examples of aerosol supply devices other than electronic cigarettes may include inhalers for ejecting a liquid, and diffusers for diffusing fragrance.

Although the foregoing description of the embodiments is directed to an exemplary configuration in which the liquid 10 is supplied toward the piezoelectric substrate 5a from below, this is not meant to be limiting. Alternatively, the liquid 10 may be configured to drip onto the piezoelectric substrate 5a from above.

Individual components of the aerosol supply device according to the present invention may not necessarily exist as independent components. For instance, the following cases may be permitted: plural components are formed as a single part; a single component is formed by plural components; a component constitutes a portion of another component; and a component and another component partially overlap.

The present application claims priority from Japanese Patent Application No. 2019-074360 filed on April 9, 2019, the entire contents of which are hereby incorporated by reference.

The embodiments described in the foregoing encompass technical ideas described below.
(1) An aerosol supply device including:
   an accommodating part configured to accommodate a liquid;
   an atomization unit configured to atomize the liquid to generate an aerosol;
   a discharge port configured to discharge the generated aerosol to an outside of the aerosol supply device; and
   a volatilization preventing part capable of opening and closing a passage through which at least one of the liquid or the aerosol flows between the accommodating part and the outside.
(2) The aerosol supply device according to (1),
   wherein a passage for the liquid is provided between the accommodating part and the atomization unit, and
   wherein the volatilization preventing part includes an open/close part capable of opening and closing the passage for the liquid.
(3) The aerosol supply device according to (1) or (2), further including
   a pump capable of supplying the liquid to the atomization unit,
   wherein the pump is a syringe pump capable of pulling back the liquid from the atomization unit toward the accommodating part.
(4) The aerosol supply device according to (3), further including
   a controller configured to control operation of the pump and operation of the volatilization preventing part,
   wherein a passage for the liquid is provided between the accommodating part and the atomization unit,
   wherein the volatilization preventing part includes an open/close part capable of opening and closing the passage for the liquid, and
   wherein the controller is configured to, in closing the passage for the liquid, control the pump such that the liquid is supplied to the atomization unit before the liquid is pulled back toward the accommodating part from the atomization unit, and cause the open/close part to close the passage for the liquid supplied to the atomization unit.
(5) The aerosol supply device according to (1),
   wherein a passage for the aerosol is provided between the atomization unit and the outside, and
   wherein the volatilization preventing part is capable of transition between a state in which the volatilization preventing part closes the passage for the aerosol, and a state in which the volatilization preventing part opens the passage for the aerosol.
(6) The aerosol supply device according to (5), wherein the volatilization preventing part is a valve element configured to close the passage for the aerosol in a first state, and open the passage for the aerosol in a second state in which a pressure condition has changed due to suction applied by a user.
(7) The aerosol supply device according to (5) or (6),
   wherein the atomization unit includes
      a substrate including a storing part to store the liquid, and a piezoelectric element, and
      an electrode disposed in contact with the substrate, the electrode being configured to supply a surface acoustic wave to the liquid stored in the storing part disposed on the substrate, and
   wherein the volatilization preventing part is disposed between the storing part and the discharge port.
(8) The aerosol supply device according to (7),
   wherein the storing part is disposed in at least two places, and the electrode is disposed between the two places,
   wherein the passage includes a plurality of passages disposed separately, the plurality of passages being each configured to allow passage of the aerosol generated from the liquid stored in the storing part disposed in the at least two places, and
   wherein the plurality of passages disposed separately each include an asymmetric portion formed with a different curvature.
(9) The aerosol supply device according to (7),
   wherein the storing part is disposed in at least two places, and the electrode is disposed between the two places, and
   wherein the passage includes two passages disposed separately, the two passages being each configured to allow passage of the aerosol generated from the liquid stored in the storing part disposed in the at least two places, the two passages being formed symmetrically with respect to an imaginary plane passing through the discharge port, and
   wherein the volatilization preventing part is disposed in each of the two passages at an equal distance from the discharge port.
(10) The aerosol supply device according to any one of (1) to (9), further including:
   a controller configured to control operation of the volatilization preventing part; and
   a detector configured to detect a signal, the signal being associated with suction of the aerosol by a user,
   wherein the controller is configured to, in response to a signal transmitted from the detector and associated with start of suction of the aerosol, control the volatilization preventing part to deactivate a function of the volatilization preventing part that prevents volatilization of the liquid.

### Reference Signs List

- 1: aerosol supply device (first embodiment)
- 2: device body (first embodiment)
- 3: mouthpiece
3a discharge port
- 4: cartridge (accommodating part)
4a body part
4b base plate
4c through-hole
4d sliding block
- 5: atomization unit
5a piezoelectric substrate (substrate)
5b storing part
5c electrode
5d through-hole
- 6: first passage (passage for liquid)
6a tube
- 7: second passage (passage for aerosol)
- 8: volatilization preventing part
8a solenoid valve (open/close part, volatilization preventing part, first volatilization preventing part)
8b duckbill valve (valve element, volatilization preventing part, second volatilization preventing part)
- 9: pump
9a motor
9b push rod
- 10: liquid
- 11: aerosol
- 12: control board
12a controller
- 13: power board
- 14: battery
- 15: power switch
- 16: negative pressure sensor (detector)
- 17: timer
- 21: aerosol supply device (second embodiment)
- 22: device body (second embodiment)
- 23: mouthpiece
- 28b: duckbill valve (first modification)
- 38: volatilization preventing part (second modification)
38a motor
38b push rod
38c sliding part
38d stationary part

## Claims

1. An aerosol supply device comprising:
an accommodating part configured to accommodate a liquid;
an atomization unit configured to atomize the liquid to generate an aerosol;
a discharge port configured to discharge the generated aerosol to an outside of the aerosol supply device; and
a volatilization preventing part capable of opening and closing a passage through which at least one of the liquid or the aerosol flows between the accommodating part and the outside.

2. The aerosol supply device according to Claim 1,
wherein a passage for the liquid is provided between the accommodating part and the atomization unit, and
wherein the volatilization preventing part includes an open/close part capable of opening and closing the passage for the liquid.

3. The aerosol supply device according to Claim 1 or 2, further comprising
a pump capable of supplying the liquid to the atomization unit,
wherein the pump is a syringe pump capable of pulling back the liquid from the atomization unit toward the accommodating part.

4. The aerosol supply device according to Claim 3, further comprising
a controller configured to control operation of the pump and operation of the volatilization preventing part,
wherein a passage for the liquid is provided between the accommodating part and the atomization unit,
wherein the volatilization preventing part includes an open/close part capable of opening and closing the passage for the liquid, and
wherein the controller is configured to, in closing the passage for the liquid, control the pump such that the liquid is supplied to the atomization unit before the liquid is pulled back toward the accommodating part from the atomization unit, and cause the open/close part to close the passage for the liquid supplied to the atomization unit.

5. The aerosol supply device according to Claim 1,
wherein a passage for the aerosol is provided between the atomization unit and the outside, and
wherein the volatilization preventing part is capable of transition between a state in which the volatilization preventing part closes the passage for the aerosol, and a state in which the volatilization preventing part opens the passage for the aerosol.

6. The aerosol supply device according to Claim 5, wherein the volatilization preventing part is a valve element configured to close the passage for the aerosol in a first state, and open the passage for the aerosol in a second state in which a pressure condition has changed due to suction applied by a user.

7. The aerosol supply device according to Claim 5 or 6,
wherein the atomization unit includes
a substrate including a storing part to store the liquid, and a piezoelectric element, and
an electrode disposed in contact with the substrate, the electrode being configured to supply a surface acoustic wave to the liquid stored in the storing part disposed on the substrate, and
wherein the volatilization preventing part is disposed between the storing part and the discharge port.

8. The aerosol supply device according to Claim 7,
wherein the storing part is disposed in at least two places, and the electrode is disposed between the two places,
wherein the passage comprises a plurality of passages disposed separately, the plurality of passages being each configured to allow passage of the aerosol generated from the liquid stored in the storing part disposed in the at least two places, and
wherein the plurality of passages disposed separately each include an asymmetric portion formed with a different curvature.

9. The aerosol supply device according to Claim 7,
wherein the storing part is disposed in at least two places, and the electrode is disposed between the two places, and
wherein the passage comprises two passages disposed separately, the two passages being each configured to allow passage of the aerosol generated from the liquid stored in the storing part disposed in the at least two places, the two passages being formed symmetrically with respect to an imaginary plane passing through the discharge port, and
wherein the volatilization preventing part is disposed in each of the two passages at an equal distance from the discharge port.

10. The aerosol supply device according to any one of Claims 1 to 9, further comprising:
a controller configured to control operation of the volatilization preventing part; and
a detector configured to detect a signal, the signal being associated with suction of the aerosol by a user,
wherein the controller is configured to, in response to a signal transmitted from the detector and associated with start of suction of the aerosol, control the volatilization preventing part to deactivate a function of the volatilization preventing part that prevents volatilization of the liquid.
